# EUROPEAN PATENT APPLICATION

(11) **EP 2 030 559 A1**
(43) Date of publication of application: **04.03.2009**
(21) Application number: 08015290.3
(22) Date of filing: 29.08.2008
(51) Int. Cl.: A61B 1/06, H01L 33/00, H05B 33/00

(54) **Light source apparatus, method of driving light source apparatus, and endoscope**

(30) Priority: 03.09.2007 JP 2007228366
(71) Applicant: FUJIFILM Corporation, Minato-ku Tokyo (JP)
(72) Inventor: Mizuyoshi, Akira, Ashigarakami-gun Kanagawa (JP)
(74) Representative: Klunker . Schmitt-Nilson . Hirsch

(57) **Abstract**

A light source apparatus (12) is provided with a diode as a light source (67) that emits excitation light for exciting phosphors (51) in an illuminator of an electronic endoscope. As excited, the phosphors emit fluorescent light, and the illuminator projects illumination light composed of the excitation light and the fluorescent light. The light source is driven by drive current supplied in the form of pulses in an exposure period of an imaging device of the endoscope. The number of pulses, the pulse width or the pulse amplitude is so controlled that the integral light volume and chromaticity of the illumination light are made proper regardless of individual variability in oscillation frequency of the light source.

## Description

### FIELD OF THE INVENTION

The present invention relates to a light source apparatus that produces illumination light by mixing excitation light from an excitation light source and fluorescent light from phosphors emitted as the phosphors are excited by the excitation light. The present invention relates also to a method of driving the light source apparatus, and an endoscope provided with the light source apparatus.

### BACKGROUND OF THE INVENTION

Such a white light illuminator has been developed that uses a light emitting diode or a semiconductor laser diode as a light source for generating excitation light, e.g. a blue ray, which excites phosphors to emit fluorescent light, e.g. green, yellow and red rays. The excitation light and the fluorescent light are mixed to produce the white light.

An application field of the white light illuminator is electronic endoscopes. Conventional electronic endoscopes use a xenon lamp or a metal halide lamp as their illuminator for illuminating insides of body cavities. To promote miniaturization of the endoscope and save the cost, recent trend is toward adopting the white light illuminator using the light emitting diode or the semiconductor laser diode as its light source.

As the illuminator emits the white light from a distal end of a probing portion of the endoscope, it is desirable to suppress heat generation induced by the light emission. In order to suppress the heat generation, a prior art disclosed in JPA2007-111151 suggests turning the light source on during reset pulse periods (exposure periods) of a CCD of the endoscope, an imaging device for capturing internal body sites for observation. This prior art further suggests changing time of activating the light source or intensity of the excitation light from the light source, so as to control volume of the white light.

The white light illuminator using the excitation light has a problem that a variation in oscillation wavelength of the excitation light causes a change in efficiency of light emission from the phosphors, which results in changing volume or chromaticity of the finally produced white light. Practically speaking, there is a little variation in oscillation wavelength between individual light emitting diodes or semiconductor laser diodes because of many factors on manufacture. For this reason, where the light emitting diode or the semiconductor laser diode is adopted as the light source of the electronic endoscope, captured images are adversely affected by the variation in oscillation wavelength of the light source. If the images are affected by the variation in oscillation wavelength of the light source, it is difficult to make systematic diagnoses. As for the chromaticity of the white light, if the proportion of the excitation light or blue ray increases so much that the light projected from the endoscope becomes bluish, images captured by the endoscope will get unsuitable for diagnoses.

JPA2007-142152 suggests making use of the above-described phenomenon that the volume or chromaticity of the white light change with the variation in oscillation wavelength of the excitation light. That is, this prior art changes pulse width and amplitude of a current for driving the light source so as to change the oscillation wavelength and volume of the excitation light in a manner to suppress the variation in chromaticity of the white light.

As a matter of fact, however, the oscillation wavelength of the excitation light, especially that of the semiconductor laser diode, hardly changes with a change in the driving current. Beside that, the light source of the endoscope in practice is provided with a temperature control device like a peltiert element, so as to prevent the temperature of the light source from changing with the change in the driving current and thus prevent the oscillation wavelength of the excitation light from changing with the temperature change of the light source. Therefore, the method of changing the oscillation wavelength of the excitation light by changing the driving current is not practically feasible.

### SUMMARY OF THE INVENTION

In view of the foregoing, a primary object of the present invention is to provide a light source apparatus that can keep the volume and chromaticity of illumination light proper.

Another object of the present invention is to provide a method of controlling driving the light source apparatus so as to keep the volume and chromaticity of the illumination light proper.

To achieve the above and other objects, on the assumption that illumination light is obtained by mixing light of a first wavelength band emitted from a light source and light of a second wavelength band emitted from phosphors as excited by the light of the first wavelength band, a light source apparatus of the present invention comprises a drive control device that supplies discrete drive current to the light source in a constant period, and changes the number of times of supplying the drive current or the magnitude of the drive current or the conduction time of the drive current to change the proportion of the light of the second wavelength band in the illumination light so as to adjust integral light volume and chromaticity of the illumination light in the constant period.

Preferably, the light source apparatus further comprises a memory device for storing data of a relation between the number of times of supplying the drive current and the change in integral light volume of the illumination light, and data of a relation between the magnitude or the conduction time of the drive current and the change in integral light volume and chromaticity of the illumination light, wherein the drive control device refers to the data stored in the memory device, to decide how to change the number of times of supplying the drive current or the magnitude of the drive current or the conduction time of the drive current.

Preferably, the drive control device changes the interval or the duty ratio of the drive current to change the conduction time of the drive current.

The constant period may be an exposure period of a solid-state imaging device that takes an image of a subject as illuminated with the illumination light.

The light source is preferably a semiconductor laser diode emitting blue rays as the light of the first wavelength band. The phosphors preferably emit green, yellow and red rays as the light of the second wavelength band.

A control method for controlling driving a light source apparatus of the present invention, which has a light source for emitting light of a first wavelength band and phosphors excited by the light of the first wavelength band to emit light of a second wavelength band, to provide illumination light by mixing the light of the first wavelength band and the light of the second wavelength band, comprises steps of:
supplying discrete drive current to the light source in a constant period; and
changing the number of times of supplying the drive current or the magnitude of the drive current or the conduction time of the drive current to change the proportion of the light of the second wavelength band in the illumination light so as to adjust integral light volume and chromaticity of the illumination light in the constant period.

According to the present invention, the light source is driven by the discrete drive current that is so controlled as to change the proportion of the fluorescent light from the phosphors to the illumination light. As the light volume and chromaticity of the illumination light changes with a change in proportion of the fluorescent light, variability in volume and chromaticity of the illumination light as induced by the individual variability of the light source can be corrected by adjusting the amount of the drive current supplied in the constant period. Thus, the illumination light is always maintained proper in volume and chromaticity.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Figure 1 is a schematic view of an electronic endoscope system;
Figure 2 is a front view of a tip of probing portion of the electronic endoscope;
Figure 3 is an enlarged sectional view of the tip of the electronic endoscope;
Figure 4 is a block diagram illustrating internal structures of a processor apparatus and a light source apparatus of the electronic endoscope system;
Figure 5 shows timing charts of reading pulses for reading charges from a solid-state imaging device, electronic shutter pulses and drive current for an excitation light source;
Figure 6 shows a timing chart illustrating a relation in magnitude between the drive current, the excitation light and fluorescent light from a phosphors as excited by the excitation light;
Figure 7 is an explanatory diagram illustrating a method of correcting the drive current by changing the number of pulses;
Figure 8 is an explanatory diagram illustrating a method of correcting the drive current by changing the pulse duty ratio;
Figure 9 is an explanatory diagram illustrating a method of correcting the drive current by changing the pulse amplitude;
Figure 10 is a table illustrating relations between the pulse number, the pulse width or the pulse amplitude of the drive current, on one hand, and integral light volume or chromaticity of illumination light, on the other hand;
Figure 11A is a graph illustrating a relation between the pulse number of the drive current and the integral light volume of the illumination light;
Figure 11B is a graph illustrating a relations between the pulse width or the pulse amplitude of the drive current, on one hand, and the integral light volume and chromaticity of the illumination light, on the other hand;
Figure 12 is a graph illustrating an example of how to correct the integral light volume and chromaticity;
Figure 13 is an explanatory diagram illustrating an example of adjusting the integral light volume and chromaticity by boosting the pulse amplitude and reducing the pulse number;
Figure 14 is an explanatory diagram illustrating an example of adjusting the integral light volume and chromaticity by boosting the pulse amplitude and reducing the pulse width;
Figure 15 is a block diagram illustrating another embodiment of the processor apparatus; and
Figure 16 is an explanatory diagram illustrating another method of adjusting the integral light volume and chromaticity of the illumination light.

### DESCRIPTION OF THE PREFERRED EMBODIMENTS

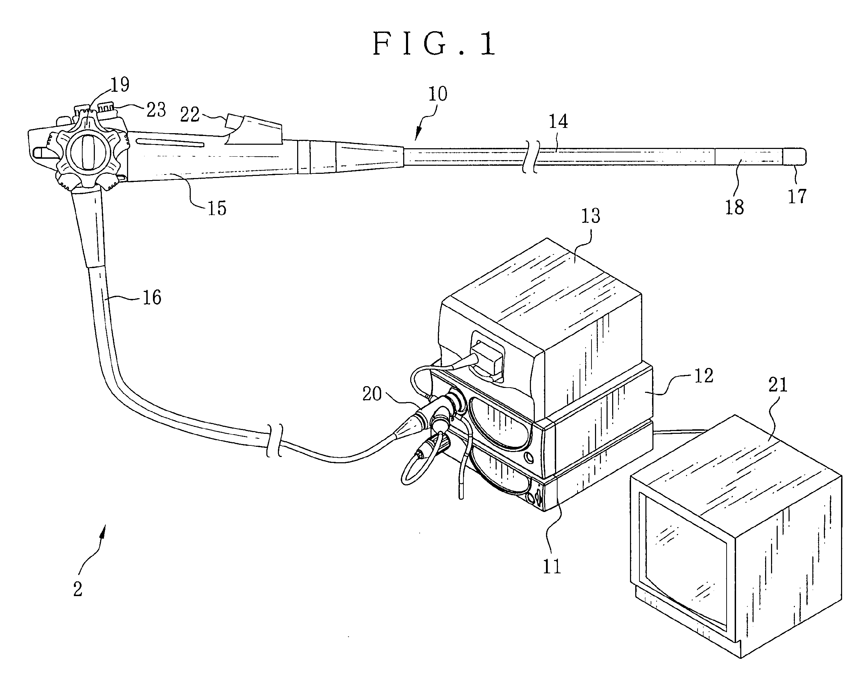

Fig.1 shows an electronic endoscope system 2 that consists of an electronic endoscope 10, a processor 11, a light source apparatus 12 and a gas/ water feeder 13. The electronic endoscope 10 is provided with a flexible probing portion 14 that is inserted into body cavities, a handling portion 15 that is joined to a base end of the probing portion 14 and a universal code 16 that is connected to the processor apparatus 11 and the light source apparatus 12.

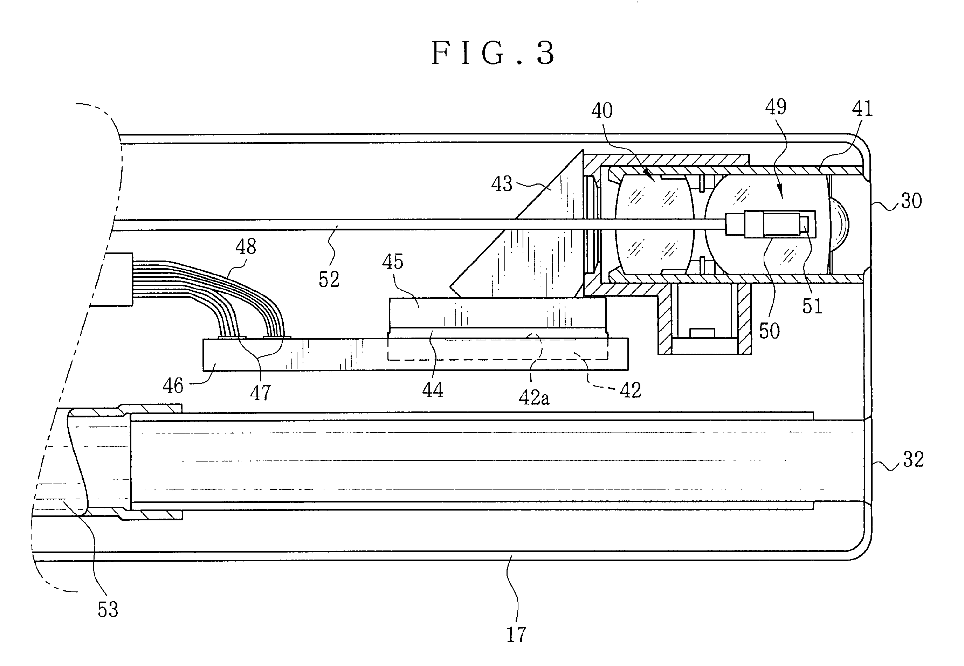

In a tip 17 of the probing portion 14 is integral a solid state imaging device 42, which images insides of the body cavities, as set forth later in Fig. 3. Behind the tip 17 is mounted a curving portion 18, which consists of serially linked segments. The curving portion 18 may curve in any directions to direct the tip 17 toward anywhere inside the body cavity. To curve the curving portion 18, an angle knob 19 of the handling portion 15 is operated to push or pull some wires that extend through inside of the probing portion 14.

A base end of the universal code 16 is connected to a connector 20, which is of a complex type and the processor apparatus 11, the light source apparatus 12 and the airing/watering apparatus 13 are individually connected to the connector 20. An image signal output from the imaging device 42 is fed through the universal code 16 and the connector 20 into the processor apparatus 11.

The processor apparatus 11 processes the image signal to convert them into picture signal, and sends a drive control signal to the imaging device 42, to control driving the imaging device 42. The picture signal is served to display an image captured by the electronic endoscope 10, hereinafter called the endoscopic image, on a monitor 21 that is cable-connected to the processor apparatus 11. The processor apparatus 11 is electrically connected to the light source apparatus 12 and the airing/watering apparatus 13, so as to control the overall operation of the electronic endoscope system 2 systematically.

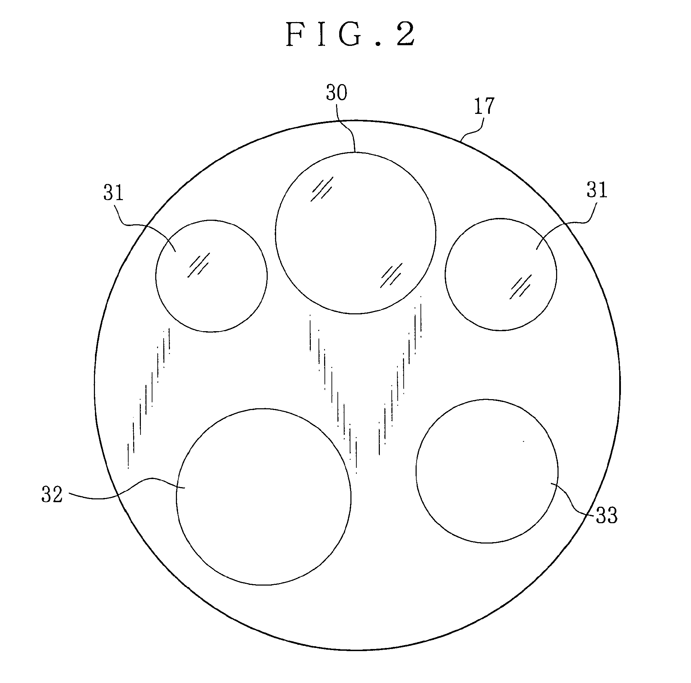

Fig. 2 shows a face end of the tip portion 17 of the electronic endoscope 10, having an observation window 30, a couple of lighting windows 31, an operation tool outlet 32 and an airing/watering nozzle 33. The observation window 30 is located at an eccentric portion, and the lighting windows 31 are arranged symmetrically on opposite sides of the observation window 30. Through the lighting windows 31, the illuminant or white light from the light source apparatus 12 is projected toward the internal body site for observation. The operation tool outlet 32 is connected through a channel 53, which is conducted along inside of the probing portion 14, as shown in Fig. 3, to an operation tool inlet 22 that is provided in the handling portion 15, as shown in Fig.1. Through the inlet 22, operation tools for medical procedures such as those having forceps, an injection needle or a high-frequency knife at their distal ends may be inserted into the channel 53, so that the distal end of the inserted operation tool protrudes out through the operation tool outlet 32. The airing/watering nozzle 33 is to eject water for washing the observation window 30 or the body cavity, or eject the air toward the observation window 30 or the body cavity. The air or water is supplied from the airing/watering apparatus 13 to the airing/watering nozzle 33 in response to an operation on an airing/watering button 23 (see Fig.1) of the handling portion 15.

Referring to Fig.3, a lens barrel 41 is placed behind the observation window 30, holding objective optics 40 therein so that an optical axis of the objective optics 40 is parallel to a center axis of the tip 17. The objective optics 40 takes an optical image of the internal body site for observation. The optical image taken through the objective optics 40 is conducted to the solid state imaging device 42, after the optical axis is bent substantially orthogonally through a prism 43 that is placed at a rear end of the lens barrel 41.

For example, the imaging device 42 is an interline CCD, and is formed as a bear chip having an imaging surface 42a on an obverse surface. The imaging surface 42a of the imaging device 42 is opposed to an output surface of the prism 43, so that the optical axis of the optical image perpendicularly incidents on the imaging surface 42a. The imaging surface 42a is covered with a rectangular cover glass plate 45 that is put on the imaging surface 42a through a rectangular spacer frame 44. The imaging device 42, the spacer 44 and the cover glass 45 are assembled together while being bonded with an adhesive agent. Thus, the imaging device 42 is held in a space surrounded and sealed by the spacer 44 and the cover glass 45, to be proofed against dusts and water.

A circuit board 46 is mounted on a bottom of the imaging device 42. The circuit board 46 holds the imaging device 42 to cover the bottom and sides of the imaging device 42. On the circuit board 46 are mounted a circuit for transmitting a drive signal for driving the imaging device 42 and other circuits. A rear end of the circuit board 46 extends toward inside of the probing portion 14, and several input-output terminals 47 are mounted on the rear end. To the input-output terminals 47, signal lines 48 of the universal code 16 are soldered, through which various signals are exchanged between the processor apparatus 11 and the imaging device 42.

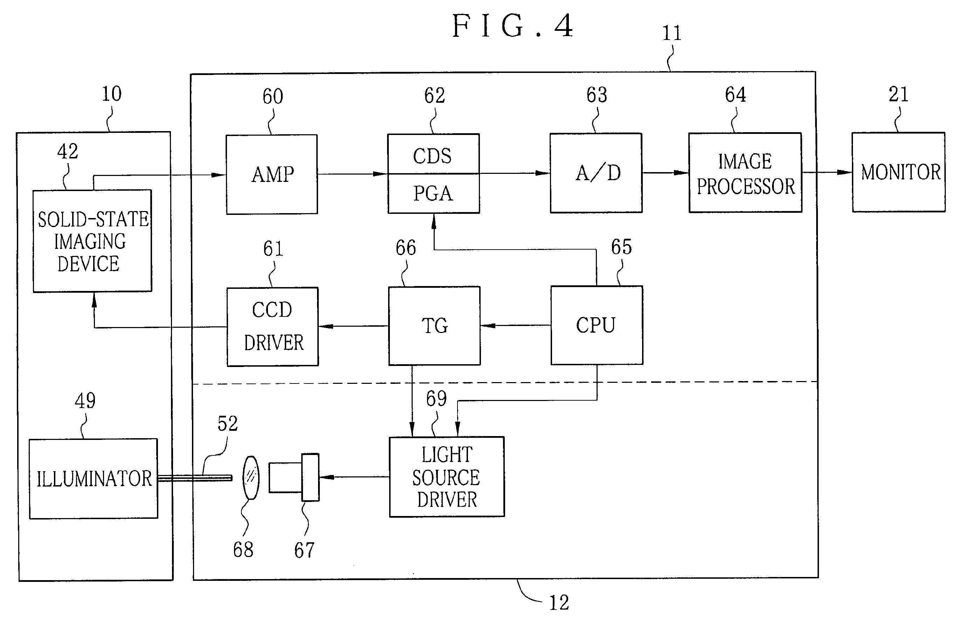

On the other hand, an illuminator 49 is mounted behind each lighting windows 31. The illuminator 49 consists of a cover member 50 and phosphors 51 that are mounted inside the cover member 50. The phosphors 51 emit light of a broad wavelength band from green to yellow and red, as it is excited by a component of blue excitation light, which is generated from a light source 67 (see Fig.4) of the light source apparatus 12 and is conducted through an optical fiber 52 to the illuminator 49. For example, the optical fibers are made of crystals. Then the blue excitation light is mixed with the light from the phosphors 51, to be converted into light of a wavelength band for white light.

The illuminator 49 is described in detail in JPA2005-205195 or "Development of high-luminance white light source using GaN-based light emitting devices", written by Yukio NARIKAWA etc. in OYO BUTURI Vol.74, No.11, p.1423, published in 2005, a membership journal of the Japan Society of Applied Physics. The phosphors 51 may be formed from phosphoric materials into a plate, or a glass kneaded with phosphors. The phosphors 51 may also be made by coating an internal surface of the cover 50 with phosphors. As the phosphoric material available for this purpose, we can refer to yellow-emitting persistent photoluminescent phosphors using silicate as base material, disclosed for example in JPA 2000-345152, and green-emitting sialon phosphors, disclosed for example in "Characterization and properties of green-emitting β-Sialon: Eu2+ powder phosphors for white light emitting diodes" written by Naoto HIROSAKI etc., 2005, Applied Physics Letters, 86, 211905.

As shown in Fig.4, the imaging device 42 is connected to an amplifier (AMP) 60 and a CCD driver 61, which are provided in the processor apparatus 11. The amplifier 60 amplifies the image signal output from the imaging device 42 with a predetermined gain, and outputs the amplified image signal to a collated double sampling/programmable gain amplifier (CDS/PGA) 62.

The CDS/PGA 62 converts the amplified image signal to analog RGB image data that exactly corresponds to charges accumulated in individual cells of the imaging device 42, amplifies the image data, and outputs the amplified image data to an A/D converter 63. The A/D converter 63 digitizes the image data, and outputs it to an image processor 64. The image processor 64 processes the digital image data so as to output an image of the internal body site on the monitor 21.

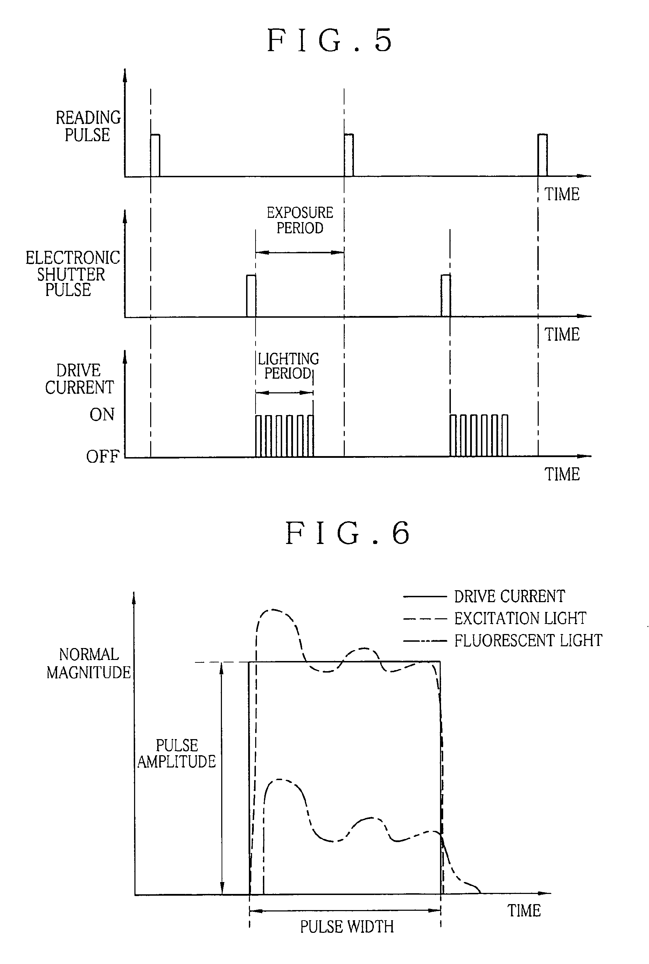

The CCD driver 61 is connected to a timing generator (TG) 66 that is controlled by a CPU 65. Based on timing signals (clock pulses) from the timing generator 66, the CCD driver 61 controls timing of reading the image signal or the accumulated charges from the imaging device 42, as well as shutter speed of an electronic shutter of the imaging device 42, as will be described with reference to Fig.5.

In the light source apparatus 12 are mounted a light source 67 for supplying an excitation light, a convergent lens 68 for converging the excitation light, and a light source driver 69 for driving the light source 67. The excitation light from the light source 67 is converged through the convergent lens 68 and conducted through the optical fiber 52 to the illuminator 49. In the present embodiment, a blue light emitting diode (LED) or a laser diode (LD), e.g. GaN-based LD, having an oscillating wavelength of 405 nm or 445 nm is used as the light source 67. Although it is not shown in the drawings, a thermostat like a peltiert element is mounted to the light source 67, to adjust the temperature of the light source 67 so that the oscillation wavelength of the excitation light is maintained approximately constant, preferably within a fluctuation range of ±2nm.

The light source driver 69 is connected to the CPU 65 and the timing generator 66. As shown in Fig.5, the light source driver 69 supplies a drive current or drive pulses under the control of the CPU 65, in an exposure period that is defined by a reading pulse for controlling the timing of reading the image signal or charges from the imaging device 42, and an electronic shutter pulse. For example, the reading pulse is generated at an interval of about 16. 7 ms (60 frame per second), and the exposure period is 12 ms.

It is known in the art that semiconductor laser diodes like GaN-based laser diodes have individual variability in oscillation wavelength of their excitation light. The individual variability in oscillation wavelength causes variability in light volume and spectral characteristics of the phosphors 51 if the light source 67 is driven uniformly by the same drive current. As a result, the integral light volume of the illumination light in the exposure period as well as the chromaticity of the illumination light, i.e. the light emission spectrum or spectral distribution of the illumination light, varies from one electronic endoscope to another. For this reason, the drive current supplied from the light source driver 69 to the light source 67 is so adjusted as to correct the variation in integral light volume and chromaticity of the illumination light, as caused by the variation in oscillation wavelength of the excitation light. Thereby, the integral light volume and chromaticity of the illumination light are adjusted to be in a suitable range in spite of the individual variability of the light source 67. The adjustment is carried out before the shipment of the electronic endoscope 10, by way of experiments and simulations.

As shown in Fig.6, the volumes of the excitation light from the light source 67 and the fluorescent light from the phosphors 51 change with time in relation to the magnitude of the drive current. That is, the excitation light is turned on and off at about the same times as leading and trailing edges of the drive current respectively. On the other hand, the fluorescent light begins to emit with a time lag from the start of emission of the excitation light, and continues emitting for a while after extinction of the excitation light, which is called afterglow. Although it is not shown in the drawings, the light volumes of the excitation light and the fluorescent light change with the magnitude or pulse amplitude of the drive current. Especially the volume of the excitation light increases with an increase in pulse amplitude of the drive current.

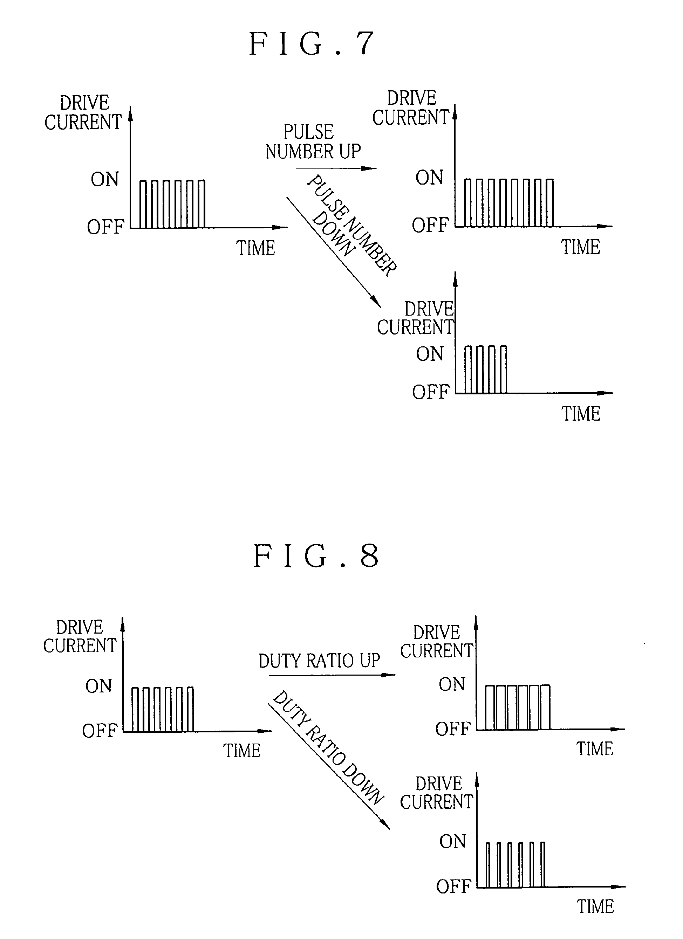

Therefore, as shown in Fig.7, where the pulse amplitude of the drive current and the conducting time or pulse width of the drive current are maintained constant, the integral light volume of the illumination light in the exposure period changes simply with the number of pulses of the drive current in the exposure period. Since the ratio of the excitation light volume to the fluorescent light volume is constant at each pulse, the chromaticity does not change with the number of pulses.

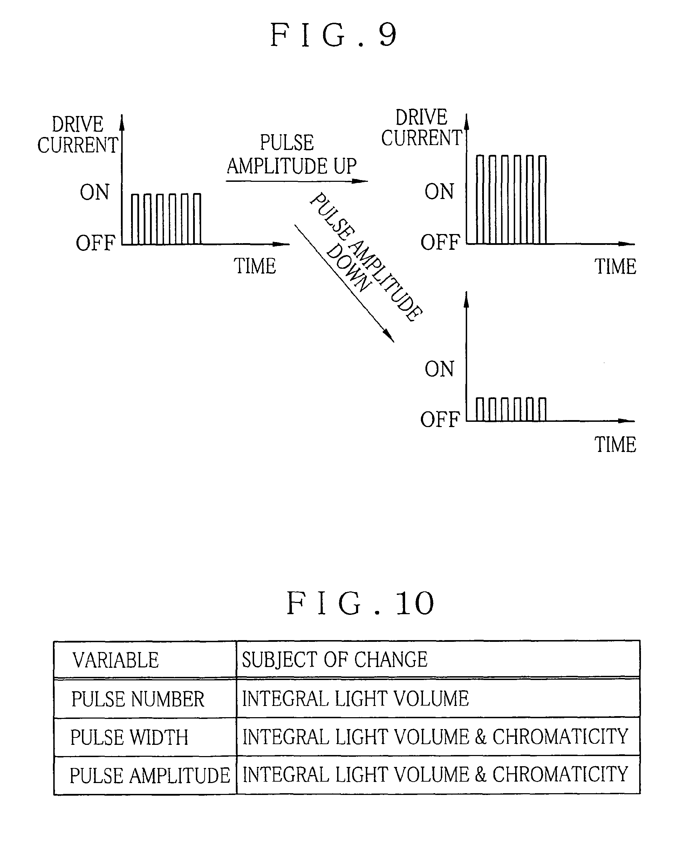

On the other hand, as shown in Fig.8, where the pulse amplitude and the pulse number are constant, and the pulse width is changed, the integral light volume of the illumination light in the exposure period changes with the pulse width. Alternatively, as shown in Fig.9, where the pulse number and the pulse width are constant, and the pulse amplitude is changed, the integral light volume of the illumination light in the exposure period changes with the pulse amplitude. In these cases, since the magnitude of the drive current has such relation as shown in Fig.6 to the volume change with time of the excitation light from the light source 67 and that of the fluorescent light from the phosphors 51, the ratio of the excitation light volume to the fluorescent light volume changes at each pulse, the chromaticity changes simultaneously with the integral light volume. Note that the pulse width is changed by changing either the pulse interval or the pulse duty ratio, that is, the ON/OFF ratio of the drive current in one interval. In the illustrated embodiment, the duty ratio is changed to change the pulse width.

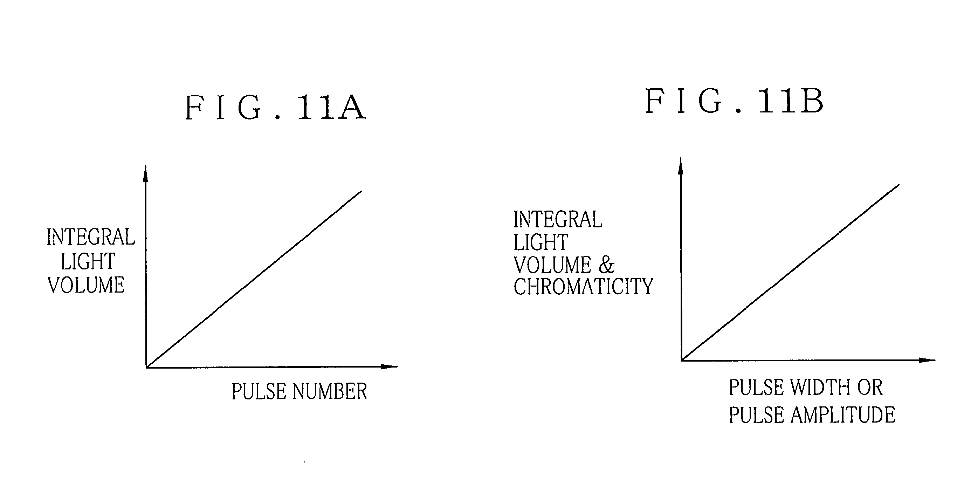

In conclusion, as shown in Fig. 10, the change in pulse number changes the integral light volume, the change in pulse width and the change in pulse amplitude change the integral light volume and the chromaticity. Accordingly, as shown in Fig.11, the relation between the pulse number and the integral light volume, and the relation between the pulse width or the pulse amplitude and the integral light volume and the chromaticity can be approximately expressed as a constantly increasing linear factor.

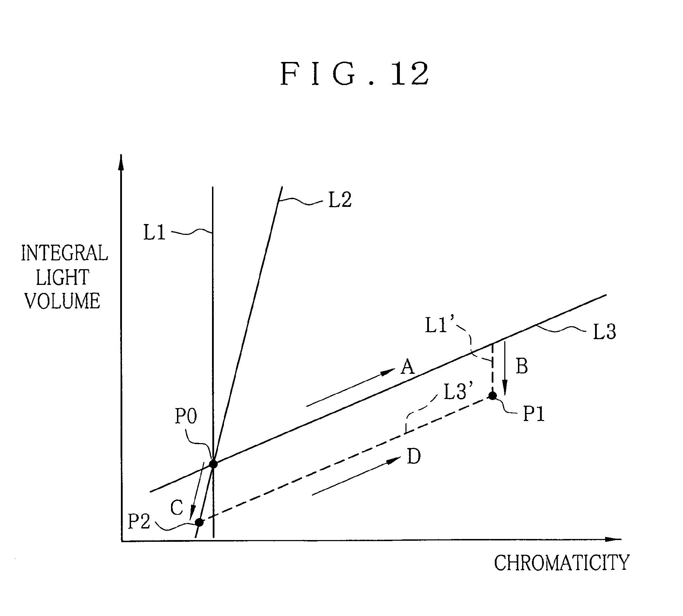

As the subject of correction, the integral light volume and the chromaticity are assumed to be vertical and horizontal axes of a coordinate space as shown in Fig.12. A point P0 represents values of the integral light volume and the chromaticity of the illumination light as generated while the light source 67 is being driven by the drive current in a default or uncorrected condition. A straight line L1 represents the change in the integral light volume that is caused by the change in the pulse number. Since the change in the pulse number changes the integral light volume only, the line L1 is parallel to the vertical line. A straight line L2 represents the change in the integral light volume and the chromaticity from the point P0 as caused by the change in the pulse width from the default pulse width of the drive current. A straight line L3 represents the change in the integral light volume and the chromaticity from the point P0 as caused by the change in the pulse amplitude from the default pulse amplitude of the drive current. A point P1 represents set values of the integral light volume and the chromaticity of the illumination light.

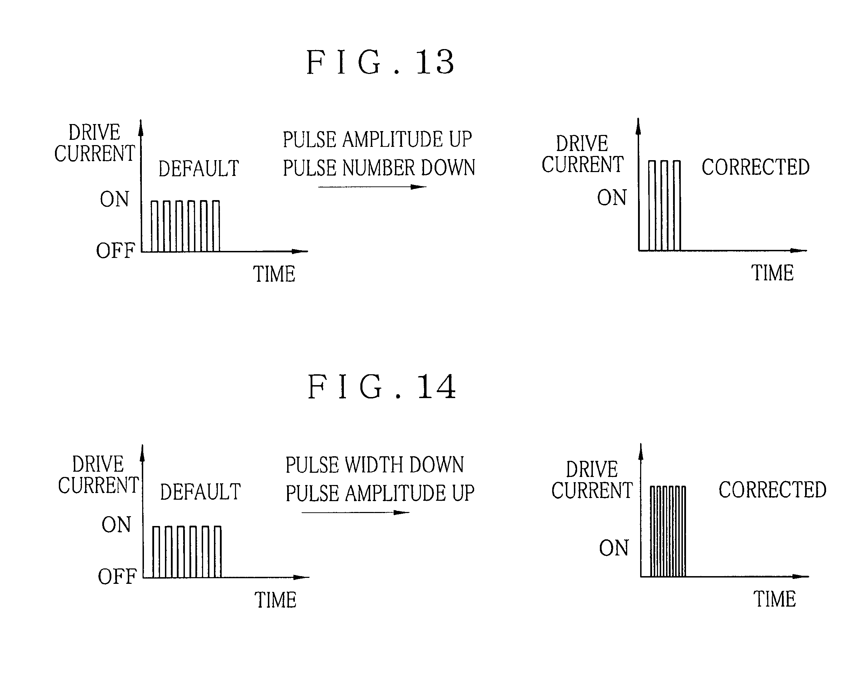

As a way to correct the integral light volume and the chromaticity from the values at the point P0 to the set values at the point P1, the pulse amplitude is boosted up as implied by an arrow A, so that the chromaticity gets to the same value as the point P1. Thereafter, as implied by an arrow B, the pulse number is reduced to reduce the integral light volume to the same value as the point P1, wherein the integral light volume is reduced along a straight line L1' that is parallel to the line L1 and cross over the point P1. Fig.13 shows this method as pulse patterns of the drive current.

Another way to correct the integral light volume and the chromaticity from the point P0 to the set point P1 is that the pulse width is reduced as implied by an arrow C, to reduce the integral light value and the chromaticity along the line L2, so that they gets to the same values as a point P2 where the line L2 crosses a line L3' , which is parallel to the line L3 and crosses over the point P1. Thereafter, as implied by an arrow D, the pulse amplitude is boosted up so that the integral light volume and the chromaticity increase along the line L3' and get to the same values as the point P1. Fig. 14 shows the second method as pulse patterns of the drive current.

Besides the above two methods, it is possible to correct the integral light volume and the chromaticity from the point P0 to the set point P1 by reducing the pulse number to reduce the integral light volume to a point where the lines L1 and L3' cross each other and boosting up the pulse amplitude, or by boosting up the pulse amplitude and thereafter reducing the pulse width.

As set forth above, the integral light volume and the chromaticity can be corrected to the set values by reducing or increasing a complex of the pulse number, width and amplitude. But the correctable range has a limit because there are upper and lower limits of these parameters. For example, the upper and lower limits of the pulse amplitude are defined by a tolerable range of the light source 67 to the drive current. If the point P1 is beyond the correctable range to the point P0, the integral light volume and the chromaticity are corrected to be the nearest point in the correctable range to the point P1.

Although the point P1 does not cross any of the lines L1 to L3 in the example shown in Fig.12, the point P1 can be on any of the lines L1 to L3. In that case, among the pulse number, the pulse width and the pulse amplitude, only one factor has to change that corresponds to the line on which the point P1 exists. Note that the lines L2 and L3 can be respectively drawn with the same inclinations at an arbitrary point other than the point P0, if the oscillation wavelength of the excitation light is constant. In other words, if the oscillation wavelength of the excitation light changes, the respective inclinations of the lines L2 and L3 change. That is, the lines L2 and L3 represent the change in integral light volume and the change in chromaticity from the arbitrary point. For the convenience of the explanation, the changes in integral light volume and chromaticity as represented the lines L2 and L3 are simplified, and the lines L2 and L3 do not represent the actual changes in integral light volume and chromaticity.

When the electronic endoscope system 2 is served to observe an internal cavity, the respective apparatuses 11 to 13 of the electronic endoscope 10 are powered on, and the probing portion 14 is inserted into the internal cavity, so that the light source apparatus 12 illuminates the inside of the internal cavity and the imaging device 42 takes images from the internal cavity. The images taken by the imaging device 42 are observable on the monitor 21.

The excitation light from the light source 67 of the light source apparatus 12 is converged through the convergent lens 68 and introduced into the optical fiber 52, through which the excitation light is conducted to the illuminator 49. A fraction of the excitation light as conducted through the optical fiber 52 excites the phosphors 51 to emit light of a broad wavelength band from green to yellow or red. Thereby, the blue excitation light from the light source 67 is converted in wavelength band to white light, so the white light is projected from the lighting window 31 toward the body site to observe.

The light source 67 is driven by the drive current from the light source driver 69. The drive current is supplied in the exposure period that is defined by the reading pulse for reading the image signal or accumulated charges from the imaging device 42 and the electronic shutter pulse. The drive current is so adjusted that the integral light volume and the chromaticity of the illumination light are made proper regardless of the individual variability of the light source 67. For example, if the oscillation wavelength of the excitation light deviates from a normal value to the shorter wavelength side, which causes a volume reduction of the fluorescent light from the phosphors 51, the pulse width or the pulse amplitude is reduced to raise the proportion of the fluorescent light in the illumination light. Thus, unevenness in integral light volume and chromaticity of the illumination light, which would result from the individual variability of the light source 67, are corrected, so that the body site to observe is irradiated with the illumination light suitable for endoscopic diagnoses.

As described so far, the light source 67 is driven by the drive current that is supplied as pulses, and the pulse number, the pulse width or the pulse amplitude is raised or reduced so that the integral light volume and the chromaticity of the illumination light are made proper regardless of the individual variability of the light source 67. Thereby, the tolerable range of the individual variability of the light source 67 is widened, so the yield of the light source 67 is improved. Moreover, diagnoses will be made under the proper illumination light.

As the illumination light for endoscopic diagnoses, white light with a slight tinge of red is desirable. Where the drive current is not supplied as pulses, it is necessary to raise the proportion of red-emitting phosphoric materials. Because the red-emitting phosphoric materials are generally inferior in conversion efficiency, they cause heat radiation and raise the temperature of the tip 17, which can cause a low-temperature burn on the body cavity. On the contrary, according to the present invention, since the light source 67 is driven by the drive pulses, the light volume of the fluorescent light increases because of the afterglow, so the proportion of the fluorescent light in the illumination light is relatively high as compared to the case where the drive current is not supplied as pulses. Therefore, it is easy to produce the reddish illumination light without raising the amount of use of the red-emitting phosphoric materials. Consequently, the tip 17 is less heated in comparison with the case where the drive current for the light source 67 is not supplied as pulses.

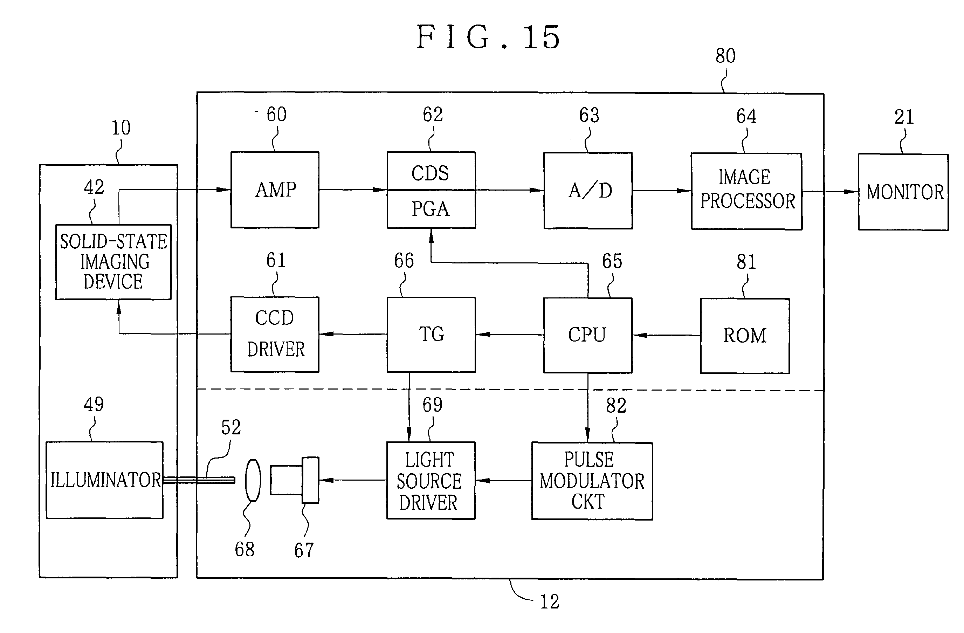

In the above-described embodiment, the drive current is previously adjusted to the oscillation wavelength of the individual light source 67 of the light source apparatus 12, so that the integral light volume and the chromaticity of the illumination light of the illumination light get proper. It is also possible to adjust the drive current afterward. As seen from the explanation referring to Fig.12, if the relation between the pulse number and the integral light volume, and the relation between the pulse width and the integral light volume and the chromaticity, or the relation between the pulse amplitude and the integral light volume and the chromaticity are known, it is apparent from the integral light volume and the chromaticity at the default drive current how to change the pulse number, the pulse width or the pulse amplitude, in order to correct the integral light volume and the chromaticity to their set values. Therefore, as shown in Fig.15, a processor apparatus 80 may be provided with a ROM 81 storing data of relations between the pulse number and the integral light volume, between the pulse width or the pulse amplitude, on one hand, and the integral light volume and the chromaticity, on the other hand. With reference to the data stored in the ROM 81, the pulse number, the pulse width or the pulse amplitude of the drive current are adjusted to make the integral light volume and the chromaticity of the illumination light proper. In that case, data of the integral light volume and the chromaticity at the default drive current may be input through a not-shown operating section of the processor apparatus 80. The pulse number, the pulse width or the pulse amplitude of the drive current may be adjusted in a pulse modulator circuit 82.

This embodiment makes it possible to correct the integral light volume and the chromaticity even after the phosphors 51 or the light source 67 is changed for some reason, such as because the component gets wrong or because the specification has changed. Therefore, it is possible to make the tip 17 removable and the phosphors 51 interchangeable. It is also possible to integrate the light source 67 and the convergent lens 68 into an interchangeable unit. Moreover, the operator may change the set values of the integral light volume and the chromaticity appropriately.

To correct the integral light volume and the chromaticity so as to cancel the individual variability in oscillation wavelength of the light source, the drive current may also be corrected in a manner as shown in Fig. 16, wherein the drive current is conducted with lower amplitude in intermissions between the default pulses, i.e. during OFF-periods of the default drive current. Thereby, the ratio of the excitation light to the fluorescent light is changed.

Although the embodiments of the present invention have been described with reference to the electronic endoscope 10, the present invention is applicable to any other endoscopes including an ultrasonic endoscope that is integral with an ultrasonic probe. The light source apparatus of the present invention may also be mounted in other equipments than endoscopes, or may be embodied as an independent apparatus.

Thus, the present invention is not to be limited to the above embodiments but, on the contrary, various modifications will be possible without departing from the scope of claims appended hereto.

## Claims

1. A light source apparatus (12) comprising:
a light source (67) for emitting light of a first wavelength band;
phosphors (51) excited by the light of the first wavelength band to emit light of a second wavelength band;
a device (49) for providing illumination light by mixing the light of the first wavelength band and the light of the second wavelength band; and
a drive control device (65, 69) that supplies discrete drive current to said light source in a constant period, said drive control device changing the number of times of supplying the drive current or the magnitude of the drive current or the conduction time of the drive current to change the proportion of the light of the second wavelength band in the illumination light so as to adjust integral light volume and chromaticity of the illumination light in said constant period.

2. A light source apparatus as recited in claim 1, further comprising a memory device (81) for storing data of a relation between the number of times of supplying the drive current and the change in integral light volume of the illumination light, and data of a relation between the magnitude or the conduction time of the drive current and the change in integral light volume and chromaticity of the illumination light, wherein said drive control device refers to the data stored in said memory device, to decide how to change the number of times of supplying the drive current or the magnitude of the drive current or the conduction time of the drive current.

3. A light source apparatus as recited in claim 1, wherein said drive control device changes the interval or the duty ratio of the drive current to change the conduction time of the drive current.

4. A light source apparatus as recited in claim 1, wherein said constant period is an exposure period of a solid-state imaging device (42) that takes an image of a subject as illuminated with the illumination light.

5. A light source apparatus as recited in claim 1, wherein said light source (67) is a semiconductor laser diode emitting blue rays as the light of the first wavelength band.

6. A light source apparatus as recited in claim 1, wherein said phosphors (51) emit green, yellow and red rays as the light of the second wavelength band.

7. An endoscope comprising a solid-state imaging device (42) for taking an image from a body site for observation inside a body cavity and a light source apparatus (12) for illuminating said body site for observation, said light source apparatus comprising:
a light source (67) for emitting light of a first wavelength band;
phosphors (51) excited by the light of the first wavelength band to emit light of a second wavelength band;
a device (49) for providing illumination light by mixing the light of the first wavelength band and the light of the second wavelength band; and
a drive control device (65, 69) that supplies discrete drive current to said light source in a constant period, said drive control device changing the number of times of supplying the drive current or the magnitude of the drive current or the conduction time of the drive current to change the proportion of the light of the second wavelength band in the illumination light so as to adjust integral light volume and chromaticity of the illumination light in said constant period.

8. A control method for controlling driving a light source apparatus (12) that has a light source (67) for emitting light of a first wavelength band and a phosphors (51) excited by the light of the first wavelength band to emit light of a second wavelength band, and provides illumination light by mixing the light of the first wavelength band and the light of the second wavelength band, wherein said control method comprises steps of:
supplying discrete drive current to said light source in a constant period; and
changing the number of times of supplying the drive current or the magnitude of the drive current or the conduction time of the drive current to change the proportion of the light of the second wavelength band in the illumination light so as to adjust integral light volume and chromaticity of the illumination light in said constant period.

9. A control method as recited in claim 8, wherein the conduction time of the drive current is changed by changing the interval or the duty ratio of the drive current.

10. A control method as recited in claim 8, wherein said constant period is an exposure period of a solid-state imaging device (42) that takes an image of a subject as illuminated with the illumination light.
